Europäisches Patentamt

European Patent Office

Office européen des brevets

(11) Publication number: **0 264 479**
**A1**

## (12) EUROPEAN PATENT APPLICATION

(21) Application number: **86114680.1**

(22) Date of filing: **22.10.86**

(51) Int. Cl.⁴: **A61B 5/10**

(43) Date of publication of application:
**27.04.88 Bulletin 88/17**

(84) Designated Contracting States:
**DE FR GB IT**

(71) Applicant: **FUKUDA DENSHI CO., LTD.**
**39-4, Hongo 3-chome Bunkyo-ku**
**Tokyo 113(JP)**

(72) Inventor: **Muraki, Yosiya**
**852, Shimoururudo**
**Hasuda-shi Saitama-ken(JP)**
Inventor: **Takahashi, Akinori**
**5-16-36, Mihara**
**Asaka-shi Saitama-ken(JP)**

(74) Representative: **Patentanwälte TER MEER -**
**MÜLLER - STEINMEISTER**
**Mauerkircherstrasse 45**
**D-8000 München 80(DE)**

(54) **Breathing detection air bag.**

(57) A breathing detection air bag is disclosed, which comprises a sponge body (2) capable of expansion and contraction in response to contractive and expansive motions of a pectoral or abdominal part of a man, a sponge cover (3) enclosing the sponge body for preventing air contained in the poses of the sponge body from escaping therefrom, a flange (4) provided integrally with the edge of the sponge cover, and an air guide tube (5) for guiding an air stream caused with expansion or contraction of the sponge body.

# FIG. 5

## BREATHING DETECTION AIR BAG

This invention relates to an air bag used for detecting the status of breathing of a man.

Heretofore, electrocardiographs have been effectively utilized to determine the health condition of patients. It is necessary to have knowledge of the condition of the breathing organ, i.e., lungs, of a patient who has a breathing disorder. The condition of the breathing organ can be determined by grasping the status of breathing, e.g., the number of breathings per unit time, waveform of breathing, etc.

The pectoral or abdominal part of a man is moved as he or she breathes. This means that the pectoral or abdominal part represents the status of breathing. Therefore, if the movement of the pectoral or abdominal part can be grasped by some or other means, the condition of the breathing organ, i.e., the lungs, can be determined by detecting and recording the status of breathing.

This is very effective as a blooding-free method of monitoring the breathing at the time of an actual surgical operation so long as there is no need of having information of the inside of the lungs.

From this viewpoint, the monitoring of the status of breathing of a patient has heretofore been performed by using an impedance system or a nasal foramen thermistor system. In the former system, the status of breathing is determined through measurement of the impedance of the surface of the patient's body. The impedance noted above is subject to changes with the breathing motion. The status of breathing is thus determined from the impedance changes.

In the latter system, a small thermometer is fitted in the nasal foramen. The status of breathing is determined through measurement of ambient temperature changes which are brought about as the patient breathes through the nose.

The status of breathing of a patient can be detected by the above methods. However, with the impedance system it is difficult to detect the status of breathing accurately in case where the sensitivity to the breathing is low or when the breathing is not so deep. Further, the nasal foramen thermistor system often makes use of an electrocardiogram electrode. In this case, the position of installation of the electrode is limited.

Further, although satisfactory breathing detection sensitivity can be obtained with the nasal foramen thermistor system, it is necessary in this case to fit a thermistor (i.e., thermometer) in the nose. Doing so spoils the appearance of the patient. In addition, pain is felt when the thermistor is held fitted for long time.

An object of the invention is to overcome the drawbacks discussed above inherent in the prior art by the provision of an air bag, which can be readily installed and permits stable monitoring of the breathing.

To attain the above object of the invention, there is provided a breathing detection air bag, which comprises a sponge body capable of expansion and contraction in response to contractive and expansive motions of a pectoral or abdominal part of a main, a sponge cover enclosing the sponge body for preventing air contained in the pores of the sponge body from escaping therefrom, a flange provided integrally with the edge of the sponge cover, and an air guide tube for guiding an air stream caused with expansion or contraction of the sponge body.

Fig. 1 is a plan view showing an embodiment of the air bag according to the invention;

Fig. 2 is a perspective view showing an essential part of the air bag shown in Fig. 1;

Figs. 3 and 4 are sectional views showing the air bag of Fig. 2;

Figs. 5 and 6 are sectional views showing the air bag coupled to a detector; and

Figs. 7 and 8 are are views for explaining the use of the air bag.

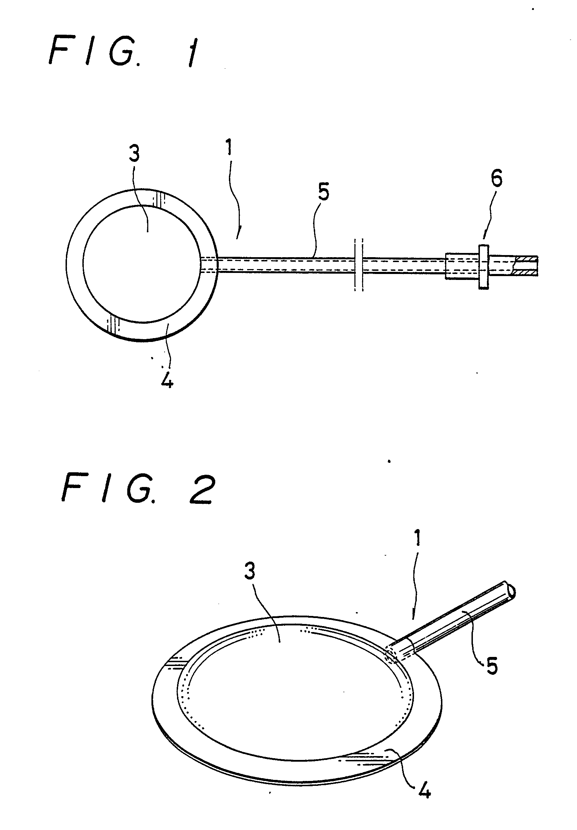

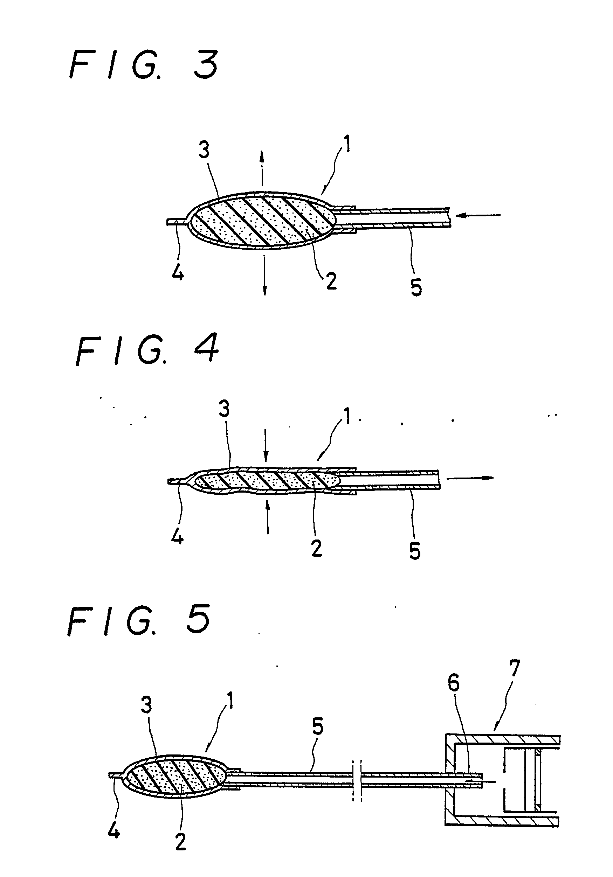

Now, the constitution and functions of the invention will be described in conjunction with an embodiment thereof with reference to the accompanying drawings. Fig. 1 is a plan view showing an embodiment of the breathing detection air bag according to the invention, Fig. 2 is a perspective view showing an essential part of the air bag shown in Fig. 1, and Fig. 3 is a sectional view showing the air bag. Referring to the Figures, there is shown an air bag generally designated at 1. The air bag 1 comprises a sponge body 2, a sponge cover 3 of vinyl chloride, a flange 4 and an air guide tube 5.

The constitution of the air bag will now be described in further detail. The sponge body 2 of the air bag 1 consists of polyurethane synthetic resin foam, which is soft and flexible and has numerous pores. The vinyl chloride sponge cover 3 entirely encloses the sponge body 2 to prevent escapement of air contained in the continous pores. The sponge body 2 enclosed in the sponge cover 3 is closely applied to the pectoral or abdominal part of the patient. In this state, it is expanded and contracted in correspondence to the movement of the pectoral or abdominal part of the patient caused with the breathing thereby. With the expansion or contraction of the sponge body 2, air is withdrawn into or forced out of the pores of the sponge body 2 enclosed in the sponge cover 3 through the air guide tube 5, as shown in Fig. 3 or 4.

The flange 4 projects outwardly from the edge of the sponge cover 3 denclosing the sponge body 2. The air guide tube 5 extends from one end of the sponge body 2, and a connector is mounted on the other end of the air guide tube 5. The flange 4 is provided in that the air bag 1 can be conveniently separated from the pectoral or abdominal part by pinching it after the air bag has been closely applied to the pectoral or abdominal part using adhesive tape.

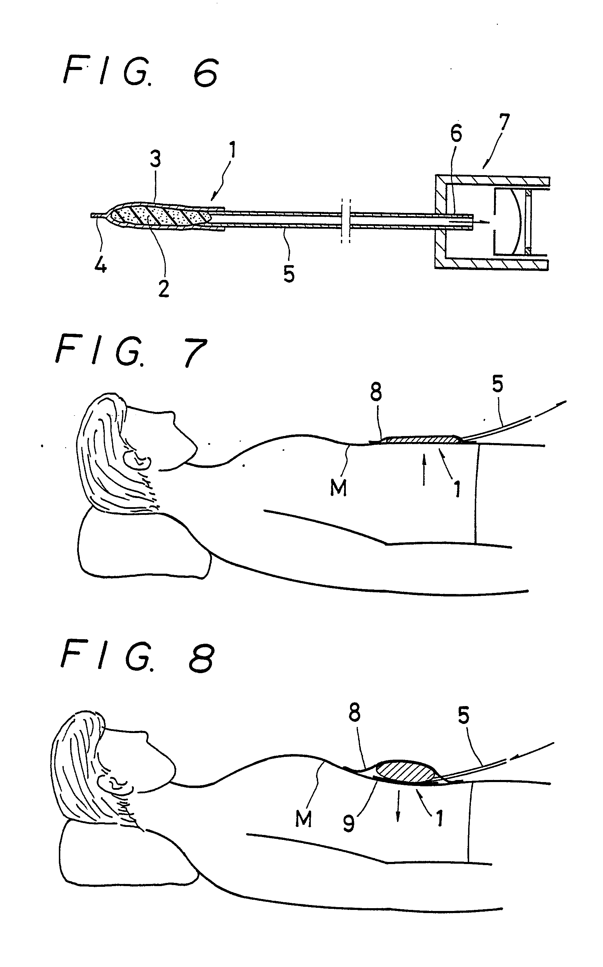

The connector 6 provided at the other end of the air guide tube permits connection of the air guide tube 5 to a detector 7, as shown in Figs 5 and and 6, which detects a movement of air due to expansion or contraction of the sponge body 2 and converts this movement of air into an electric signal. The detailed construction of the detector 7 is irrelevant to the subject matter of the invention, so its detailed description is omitted.

The air bag 1 having the above construction is used in the following way. As shown in Fig. 7, the air bag 1 is directly applied to an abdominal part M of the patient using a single side adhesive tape 8. Alternatively, as shown in Fig. 8, a double side adhesive tape 9 is first applied to the abdominal part M, the bottom of the air body 1 is bonded to the other side of the double side adhesive tape 9, and then the air bag 1 is converted with an adhesive tape 8.

The applied air bag 1 undergoes expansion and contraction with contractive and expansive motions of the abdomen caused with the breathing of the patient. When the abdomen M is expanded, the air bag 1 is urged and contracted, so that air contained in the air bag 1 is forced out through the air guide tube 5 into the detector 7, as shown in Fig. 6. In this case, the sponge body 2 is held in a contracted state. When the abdomen M is contracted, as shown in Fig. 7, the air bag 1 is expanded, so that air that has been forced into the detector 7 is returned through the air guide tube 5 into the air bag 1, as shown in Fig. 5. In this case, the sponge body 2 serves an auxiliary role with its inherent restoring force.

The air bag 1 may be used not only for the breathing detection method described above, but it may also be effectively utilized for the detection of the cervical arterial wave in close contact with the top of the cervial arterial skin and also for the detection of finger tip sphygmic wave, apex cordis pulsations, etc.

Further, while usually a single air bag 1 is used for the detection of the breathing, sphygmic waves, etc., it is also possible to apply a plurality of air bags 1 to the body surface of a patient for pneumatically or electrically processing their pressure variations (e.g., taking the sum of pressure differences) to obtain data for the detection.

As has been described in the foregoing, the air bag according to the invention is simple in construction, can be inexpensively manufactured and permits easy detection of the status of breathing.

Further, the status of breathing can be detected by merely closely applying the air bag to the pectoral or abdominal part of the patient. Further, since it is not used together with an electrocardiogram electrode, its position of installation is not limited, and its position can be freely determined.

## Claims

1. A breathing detection air bag **characterized by**
a sponge body (2) capable of expansion and contraction in response to contractive and expansive motions of a pectoral or abdominal part of a man;
a sponge cover (3) enclosing said sponge body for preventing air contained in the pores of said sponge body from excaping therefrom;
a flange (4) provided integrally with the edge of said sponge cover; and
an air guide tube (5) for guiding an air stream caused with expansion or contraction of said sponge body.

2. The breathing detection air bag according to claim 1, wherein said sponge body (2) is made of a synthetic resin.

3. The breathing detection air bag according to claim 1, wherein said sponge body (2) is made of a rubber-like material.

4. The breathing detection air bag according to claim 1, wherein said sponge cover (3) enclosing said sponge body is made of vinyl chloride.

0 264 479

Fukuda Denshi Co., Ltd.

FK014

# F I G. 1

# F I G. 2

# F I G. 3

# F I G. 4

# F I G. 5

# F I G. 6

# F I G. 7

# F I G. 8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 019 321 (PYE (ELECTRONIC PRODUCTS) LTD.) <br> * Figures 1-5; page 3, line 25 - page 6, line 24 * | 1-4 | A 61 B 5/10 |
| A | US-A-4 175 263 (TRIPLETT et al.) <br> * Figures 2-4; column 3, line 54 - column 5, line 64 * | 1-4 | |

**TECHNICAL FIELDS SEARCHED (Int. Cl.4)**

A 61 B

The present search report has been drawn up for all claims

| Place of search | Date of completion of the search | Examiner |
|---|---|---|
| THE HAGUE | 22-06-1987 | CHEN A.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82